# EUROPEAN PATENT APPLICATION

(11) **EP 4 260 892 A1**
(43) Date of publication of application: **18.10.2023**
(21) Application number: 20965111.6
(22) Date of filing: 10.12.2020
(51) Int. Cl.: A61M 15/00, A24F 40/50, A24F 40/65, A61M 11/04

(54) **INFORMATION PROCESSING DEVICE, INFORMATION PROCESSING METHOD, PROGRAM, MEDICAL DEVICE, AND INHALATION DEVICE**

(71) Applicant: Japan Tobacco Inc., Tokyo 105-6927 (JP)
(72) Inventor: OSAWA, Noriko, Tokyo 130-8603 (JP); SENJU, Masatoshi, Tokyo 130-8603 (JP)
(74) Representative: Hoffmann Eitle
(86) International application number: PCT/JP2020/046059
(87) International publication number: WO 2022/123729

(57) **Abstract**

This information processing device is provided with: a first acquisition unit that acquires, from a medical device, inhalation information relating to inhalation by a user of a drug atomized by the medical device on the basis of inhalation by the user; a first calculation unit that calculates total inhalation information obtained by summing information relating to inhalation by the user on the basis of the inhalation information acquired by the first acquisition unit; a second acquisition unit that acquires atomization information relating to the amount of atomization of the drug; a second calculation unit that calculates the amount of intake of the drug by the user using the total inhalation information calculated by the first calculation unit and the atomization information acquired by the second acquisition unit; and an output unit that outputs information relating to the amount of intake of the drug calculated by the second calculation unit.

## Description

### FIELD

The present invention relates to an information processing device, an information processing method, a program, a medical device, and an inhaler.

### BACKGROUND

In recent years, a technique for communicating medical data such as an amount of vaporized medicament with an external device in a portable medical device has been developed.

For example, Patent Document 1 discloses that data relating to a vaporized substance acquired in a medical device is transmitted externally.

### CITATION LIST

### PATENT LITERATURE

[Patent Document 1] International Publication No. 2019/191408

### SUMMARY

### TECHNICAL PROBLEM

Here, in Patent Document 1, a medical device is controlled to provide information relating to a dose of the vaporized substance to an external device. However, the vaporized substance may be dispersed in the air before being ingested by the user. Therefore, there may be a difference between the amount of the vaporized substance and the user's actual intake amount.

An object of the present invention is to provide a technique capable of more accurately calculating an intake amount of a medicament of a user in a medical inhaler.

### SOLUTION TO PROBLEM

An information processing device according to an aspect of the present invention includes: a first acquisition unit configured to acquire, based on inhalation by a user of a medicament atomized by a medical device, inhalation information relating to inhalation by the user from the medical device; a first calculation unit configured to calculate total inhalation information obtained by summing information relating to inhalation per inhalation by the user based on the inhalation information acquired by the first acquisition unit; a second acquisition unit configured to acquire atomization information relating to an atomization amount of the medicament; a second calculation unit configured to calculate an intake amount of the medicament by the user by using the total inhalation information calculated by the first calculation unit and the atomization information acquired by the second acquisition unit; and an output unit configured to output information relating to the intake amount of the medicament calculated by the second calculation unit.

An information processing method according to an aspect of the present invention includes: acquiring, based on inhalation by a user of a medicament atomized by a medical device, inhalation information relating to inhalation by the user from the medical device; calculating total inhalation information obtained by summing information relating to inhalation of each inhalation by the user based on the acquired inhalation information; acquiring atomization information relating to an atomization amount of the medicament; calculating an intake amount of the medicament by the user by using the calculated total inhalation information and the acquired atomization information; and outputting information relating to the intake amount of the calculated medicament.

A program according to an aspect of the present invention causes a computer to implement: a first acquisition unit configured to acquire, based on inhalation by a user of a medicament atomized by a medical device, inhalation information relating to inhalation by the user from the medical device; a first calculation unit configured to calculate total inhalation information obtained by summing information relating to inhalation of each inhalation by the user based on the inhalation information acquired by the first acquisition unit; a second acquisition unit configured to acquire atomization information relating to an atomization amount of the medicament; a second calculation unit configured to calculate an intake amount of the medicament by the user by using the total inhalation information calculated by the first calculation unit and the atomization information acquired by the second acquisition unit; and an output unit configured to output information relating to the intake amount of the medicament calculated by the second calculation unit.

In the present invention, the "unit" or "device" does not simply refer to a physical means, but encompasses a case where functions equipped in the "unit" or "device" are implemented by software. The functions equipped in a single "unit" or "device" may be implemented by two or more physical means or devices, and the functions of two or more "units" or "devices" may be implemented by a single physical means or device.

Also, "vaporization" or "atomization" generally refers to misting or aerosolizing a medicament.

### ADVANTAGEOUS EFFECTS OF INVENTION

According to the present invention, it is possible to provide a technique capable of more accurately calculating an intake amount of a medicament of a user in a medical inhaler.

### BRIEF DESCRIPTION OF THE DRAWINGS

FIG. 1 is a block diagram schematically showing a configuration example of an information processing system according to an embodiment of the present invention.
FIG. 2 is a schematic diagram schematically showing a configuration example of a medical inhaler according to the embodiment of the present invention.

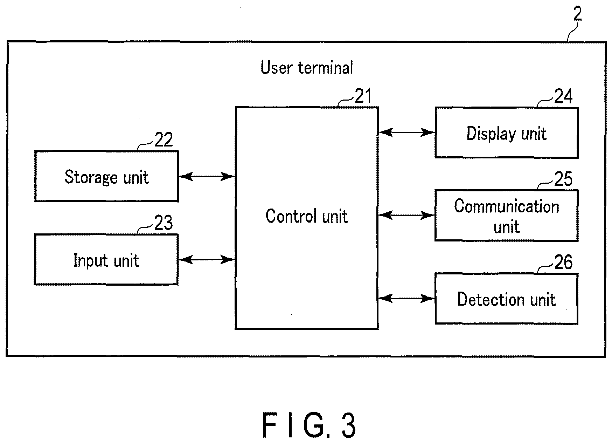

FIG. 3 is a block diagram schematically showing a configuration example of a user terminal according to the embodiment of the present invention.
FIG. 4 is a block diagram schematically showing a configuration example of an information processing server according to the embodiment of the present invention.
FIG. 5 is a diagram schematically showing a configuration example of a denaturation data DB according to the embodiment of the present invention.
FIG. 6 is a diagram schematically showing a configuration example of a particle size data DB according to the embodiment of the present invention.
FIG. 7 is a diagram schematically showing a configuration example of a delivery rate DB according to the embodiment of the present invention.
FIG. 8 is a flowchart showing an example of processing by the information processing server according to the embodiment of the present invention.
FIG. 9 is a flowchart showing an example of processing by the information processing server according to the embodiment of the present invention.
FIG. 10 is a flowchart showing an example of processing by the medical inhaler according to the embodiment of the present invention.

### DETAILED DESCRIPTION

Hereinafter, an aspect of an embodiment of the present invention will be described with reference to the accompanying drawings. In the drawings, the same constituent elements will be assigned identical reference symbols where possible, and redundant descriptions will be omitted.

### <<Configuration Example of Information Processing System>>

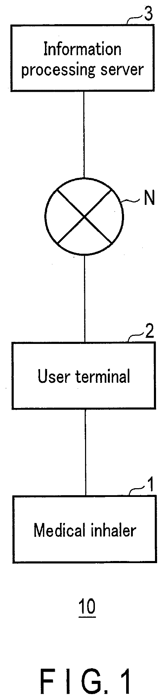

FIG. 1 is a schematic configuration diagram of an information processing system according to an embodiment of the present invention.

As shown in FIG. 1, an information processing system 10 includes, as an example, a medical inhaler 1, a user terminal 2 connected to the medical inhaler 1, and an information processing server 3 configured to enable communication with the user terminal 2 via a network N. In the following description, for example, the medical inhaler 1 and the user terminal 2 are possessed by the same user. The medical inhaler 1 is an example of a medical device or an inhaler. The information processing server 3 is an example of an information processing device. The user may be read as a patient or a person.

The information processing system 10 is what is known as a "client server system". The information processing system 10 is realized by mutual communications between the user terminal 2, which is a client, and the information processing server 3 via the network N. The network N is realized by, for example, the Internet, a network such as a mobile telephone network, a local area network (LAN), or a network that is a combination thereof.

The medical inhaler 1 is an electronic device for generating a substance to be inhaled by the user. The medical inhaler 1 is, for example, a nebulizer. Other than the above, for example, a heating stick may be inserted into the device for inhalation. A configuration example of a medical inhaler that may serve as the medical inhaler 1 will be described later.

A user terminal 2 is a portable electronic device equipped with a communication function. For example, the user terminal 2 is a smartphone, a tablet terminal, or the like. A configuration example of the user terminal 2 will be discussed later.

The information processing server 3 is a computer equipped with an information processing function. The information processing server 3 is realized by, for example, one or more (at least one) server devices. A configuration example of the information processing server 3 will be described later.

The medical inhaler 1 and the user terminal 2 are associated with each other, and mutual data transmission and reception are enabled by executing short-range wireless communications based on, for example, Bluetooth (registered trademark), Bluetooth Low Energy (BLE), etc. Data transmission and reception between the medical inhaler 1 and the user terminal 2 may be executed by any communication technology such as Wi-Fi (registered trademark), a low-power wide-area network (LPWAN), a near-field communication (NFC), or the like, aside from BLE communication technology. Also, data transmission and reception between the medical inhaler 1 and the user terminal 2 is not necessarily performed by wireless communications, and may be performed by wired communications using a Universal Serial Bus (USB), a Mini USB, a Micro USB, Lightning, or the like.

### «Configuration Example of Medical inhaler»

The medical inhaler is a device that produces a substance to be inhaled by the user. In the description that follows, it is assumed that the substance produced by the medical inhaler is an aerosol. In another case, the substance produced by the medical inhaler may be a gas.

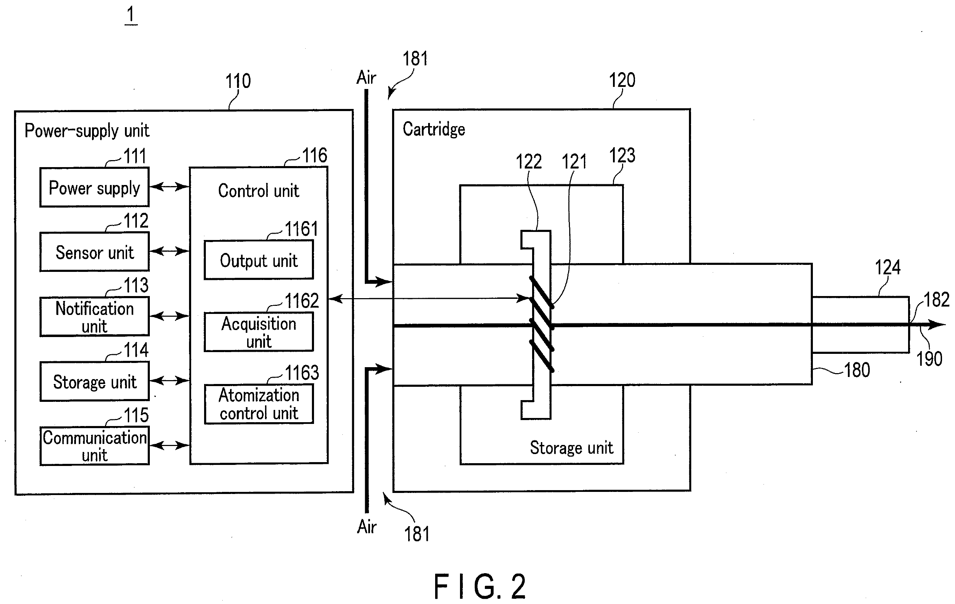

FIG. 2 is a schematic diagram schematically showing a configuration example of the medical inhaler.

As shown in FIG. 2, the medical inhaler 1 according to the present configuration example includes a power supply unit 110, a cartridge 120, and a mouthpiece 124. The power-supply unit 110 includes a power supply 111, a sensor unit 112, a notification unit 113, a storage unit 114, a communication unit 115, and a control unit 116. The cartridge 120 includes a heating unit 121, a liquid guiding unit 122, and a storage unit 123. In the cartridge 120, an airflow path 180 is formed.

The power supply 111 accumulates electric power. Based on control by the control unit 116, the power supply 111 supplies power to each constituent element of the medical inhaler 1. The power supply 111 may be configured of, for example, a chargeable battery such as a lithium-ion secondary battery.

The sensor unit 112 acquires various types of information relating to the medical inhaler 1. As an example, the sensor unit 112 is configured of a pressure sensor such as a microphone capacitor, a flowrate sensor, a voltage sensor, a temperature sensor, etc., and acquires values involved with user inhalation. For example, the sensor unit 112 may measure an inhalation volume [ml/sec] of the user's inhalation to acquire the inhalation volume. As another example, the sensor unit 112 is configured of an input device which receives an information input from the user, such as a button, a switch, etc. Furthermore, the sensor unit 112 includes a vital sensor that measures biometric data.

The notification unit 113 notifies the user of the information. The notification unit 113 is configured of, for example, a light-emitting device which emits light, a display device which displays an image, a sound output device which outputs sound, a vibration device which vibrates, etc.

The storage unit 114 stores various types of information for operation of the medical inhaler 1. The storage unit 114 is configured of, for example, a non-volatile storage medium such as a flash memory.

The communication unit 115 is a communication interface that enables communications compatible with a given wired or wireless communication standard to be performed. As such a communication standard, Wi-Fi (registered trademark), Bluetooth (registered trademark), etc. may be adopted.

The control unit 116 functions as an arithmetic processor and a controller, and controls the entire operation of the medical inhaler 1 in accordance with various programs. The control unit 116 is realized by, for example, electronic circuitry such as a central processing unit (CPU), or a microprocessor. By executing the programs stored in the storage unit 114, the control unit 116 realizes the respective units that will be discussed later, and executes a variety of operations.

The storage unit 123 stores an aerosol source. By atomizing the aerosol source, aerosol is produced. The aerosol source is, for example, a polyhydric alcohol such as glycerin and propylene glycol, a liquid such as water, and a medicinal solution containing a medicament.

The liquid guide unit 122 guides the aerosol source which is a liquid stored in the storage unit 123 from the storage unit 123, and holds the aerosol source. The liquid guide unit 122 is, for example, a wick formed by twisting a fiber material such as glass fiber or a porous material such as a porous ceramic. In this case, the aerosol source stored in the storage unit 123 is guided by the capillary effect of the wick.

The heating unit 121 heats the aerosol source, atomizes the aerosol source and produce aerosol. In the example shown in FIG. 2, the heating unit 121 is configured as a coil, and is wound around the liquid guide unit 122. When the heating unit 121 generates heat, the aerosol source held in the liquid guide unit 122 is heated and atomized, thus producing aerosol. The heating unit 121 generates heat when fed from the power supply 111. As an example, feeding may be performed when the sensor unit 112 has detected the commencement of inhaling by the user and/or the input of predetermined information. Feeding may be stopped when the sensor unit 112 has detected the termination of inhaling by the user and/or the input of predetermined information. The heating unit 121 is an example of an atomizing unit.

The airflow path 180 is a path of air inhaled by the user. The airflow path 180 has a tubular structure including, at one of both ends, an air inflow hole 181, which is an inlet of air flowing to the airflow path 180, or an air outflow hole 182, which is an outlet of air flowing from the airflow path 180. In the middle of the airflow path 180, the liquid guide unit 122 is arranged on an upstream side (the side closer to the air inflow hole 181), and the cartridge 120 is arranged on a downstream side (the side closer to the air outflow hole 182). The air flowing in from the air inflow hole 181 in accordance with user inhalation is mixed with the aerosol produced by the heating unit 121, and, as shown by an arrow 190, and is transported to the air outflow hole 182.

The mouthpiece 124 is a member which comes in contact with the user's mouth during inhaling. In the mouthpiece 124, the air outflow hole 182 is arranged. The user can introduce a mixed fluid of the aerosol and air into the oral cavity through inhalation via the mouthpiece 124 put in the user's mouth. The mouthpiece 124 is an example of an inhalation unit. The mouthpiece 124 is used to inhale the aerosol source atomized by the heating unit 121.

A configuration example of the medical inhaler 1 has been described above. As a matter of course, the configuration of the medical inhaler 1 is not limited to the above, and various configurations that will be described below as examples may be adopted.

As another example, the medical inhaler 1 may include a plurality of types of aerosol sources. Still other types of aerosol may be produced by a chemical reaction caused by a plurality of types of aerosol produced by a plurality of types of aerosol sources being mixed in the airflow path 180.

Moreover, the means for atomizing the aerosol source are not limited to heating by the heating unit 121. For example, the aerosol source may be atomized by means such as vibration atomizing or induction heating.

The medical inhaler 1 configured as described above starts measurement of biometric data based on activation, and transmits the measured biometric data to the information processing server 3 via the user terminal 2. The biometric data may be transmitted in real time, or may be transmitted at a timing at which predetermined information is transmitted. The biometric data is measured during activation of the medical inhaler 1. The biometric data is measured while the user is inhaling with the medical inhaler 1. In one example, the medical inhaler 1 begins measurement of biometric data based on activation of the medical inhaler 1 in response to an inhalation action of the medical inhaler 1 by the user. In another example, the medical inhaler 1 begins measurement of the biometric data based on an input of activation by the user using a button, a switch, or the like of the medical inhaler 1.

Here, an example of biometric data measured in the medical inhaler 1 will be described. The biometric data is data that can be measured in the medical inhaler 1 using a known technique with respect to indexes, such as pulses, perspiration, salivary components, heartbeats, respiration, words, and a grip strength. The data relating to salivary components may include data such as concentrations relating to amylase (u/L), serotonin (µg/mL), cotisol (Pmol/mL), nitric oxide metabolite (µg/g) (salivary nitrate ion). The measurement data related to salivary components may include data such as volatile sulfur compounds (ppb) (hydrogen sulfide, methyl mercaptan, and dimethyl sulfide), acidity (pH), buffer capacity (pH), white blood cells (cells/µL), protein (mg/mL), ammonia (µg/mL), and viscosity (cp) in addition to the above-mentioned data.

Each of the units realized by the above-described control unit 116 will be described.

The control unit 116 realizes an output unit 1161, an acquisition unit 1162, and an atomization control unit 1163. It can also be said that each unit is realized by processing circuitry such as a processor included in the control unit 116. Each unit can also be referred to as each function. Each unit described below can be read as the control unit 116 or processing circuitry such as a processor.

The output unit 1161 outputs various kinds of information based on the activation or the inhalation action of the medical inhaler 1 to the information processing server 3.

For example, the output unit 1161 outputs inhalation information relating to inhalation of a medicament by the user through the mouthpiece 124 to the information processing server 3. Hereinafter, the "medicament" means a liquid or medicinal solution containing a medicament. The inhalation information is information acquired based on detection of the inhalation action. The detection of the inhalation action is realized by detecting an airflow in the airflow path 180 generated by the inhalation action of the user. The inhalation information includes, for example, information relating to an inhalation volume of the user. The information relating to the inhalation volume of the user may be an inhalation volume [ml/sec] of the user per unit time or an inhalation volume [ml] per single inhalation. The medical inhaler 1 acquires information relating to the inhalation volume of the user through the pressure sensor or the flowrate sensor serving as the sensor unit 112. The inhalation information may include start information, end information, or inhalation time information. For example, the medical inhaler 1 acquires information indicating the start or end of the inhalation action in response to detection of the start or end of the inhalation action based on the inhalation action of the medical inhaler 1 by the user. The information indicating the start of the inhalation action corresponds to start information indicating the start of inhalation. The information indicating the end of the inhalation action corresponds to end information indicating the end of inhalation. The inhalation time information is information indicating an inhalation time obtained by measuring a time from the start to the end of the inhalation action.

The output unit 1161 outputs, for example, the biometric data acquired based on the activation of the medical inhaler 1. The output unit 1161 outputs, for example, temperature information. The temperature information is information indicating the temperature of the heating unit 121. The temperature of the heating unit 121 is measured by the sensor unit 112. The output unit 1161 outputs, for example, voltage information. The voltage information is information indicating the voltage of the heating unit 121. The voltage of the heating unit 121 is measured by the sensor unit 112. The notation of voltage means a voltage value. There is a correlation between the voltage of the heating unit 121 and the temperature of the heating unit 121.

The acquisition unit 1162 acquires an operation instruction of the heating unit 121 from the information processing server 3 as a response to the inhalation information output by the output unit 1161. The operation instruction includes a temperature adjustment instruction, a stop instruction, and the like. The temperature adjustment instruction is, for example, an instruction to adjust the temperature by increasing or decreasing the power supply amount of the heating unit 121. The stop instruction is, for example, an instruction to stop power supply to the heating unit 121.

The atomization control unit 1163 controls the temperature adjustment or stopping of the heating unit 121 based on the operation instruction. The atomization control unit 1163 is an example of a control unit that controls the heating unit 121.

### «Configuration Example of User Terminal»

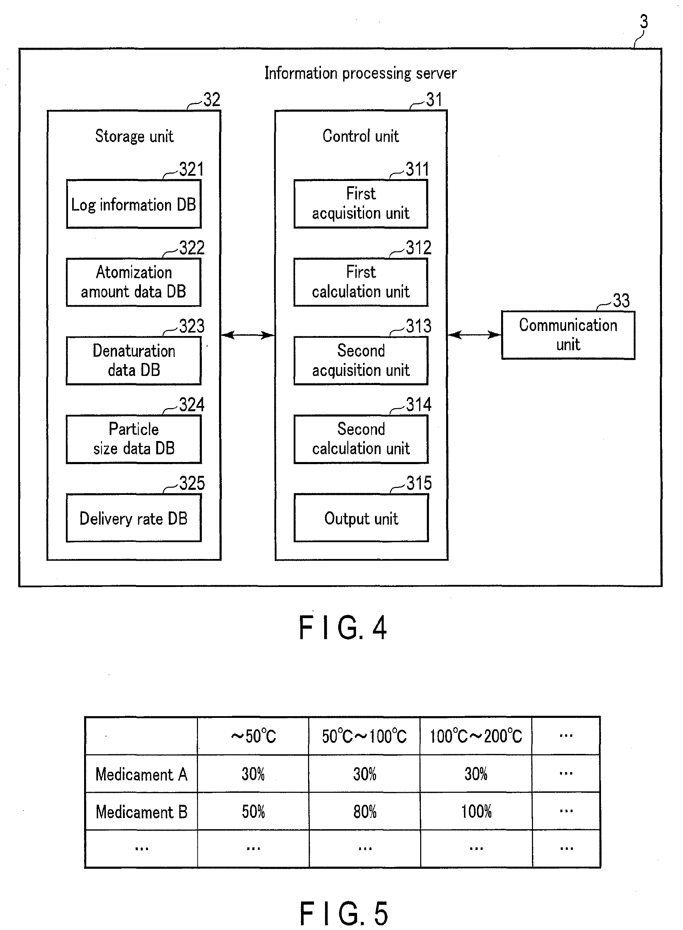

FIG. 4 is a block diagram schematically showing a configuration example of a user terminal according to the embodiment of the present invention.

The user terminal 2 includes a control unit 21, a storage unit 22, an input unit 23, a display unit 24, a communication unit 25, and a detection unit 26. These units are mutually connected via a bus line.

The control unit 21 controls the entire operation of the user terminal 2 in accordance with a program stored in the storage unit 22. The control unit 21 is configured of, for example, electronic circuitry such as a processor. The processor is, for example, a CPU or the like.

The storage unit 22 is configured of a main storage and an auxiliary storage. The main storage is configured of, for example, a volatile memory that provides a work area for the processor. The main storage is configured of, for example, a random access memory (RAM) or the like. The auxiliary storage is configured of, for example, a non-volatile memory that stores various types of information and programs for operation of the user terminal 2. The auxiliary storage is configured of, for example, a hard disk drive (HDD), a solid-state drive (SSD), or the like.

Based on the user's operation, the input unit 23 receives an instruction. The input unit 23 is configured of, for example, a keyboard, a touchpad, or the like.

The display unit 24 displays a variety of screens. The display unit 24 is configured of, for example, a liquid crystal display.

The communication unit 25 is configured of one or more communication interfaces that enable communications compatible with a given wired or wireless communication standard to be performed. The communication unit 25 includes one or more communication interfaces that enable communications to be performed between the user terminal 2 and the medical inhaler 1, as described above. The communication unit 25 includes one or more communication interfaces that enable communications to be performed between the user terminal 2 and the information processing server 3 via the network N, as described above.

The detection unit 26 is configured of a sensor that detects various types of information. The detection unit 26 includes a sensor that detects position information of the user terminal 2. For example, the position information is information indicating latitude and longitude. The sensor that detects the position information is, for example, a global positioning system (GPS) sensor.

The user terminal 2 described above cooperates with the medical inhaler 1 through activation of a specific application. As exemplified below, the user terminal 2 transmits log information including inhalation information to the information processing server 3. For example, the log information may include time information, position information, and the like relating to acquisition of the inhalation information. The time information relating to the acquisition of the inhalation information is, for example, information indicating the time at which the user terminal 2 acquires the inhalation information. The position information relating to the acquisition of the inhalation information is, for example, information indicating the position of the user terminal 2 at the time when the user terminal 2 acquires the inhalation information.

The user terminal 2 transmits the log information to the information processing server 3 based on the inhalation action of the medical inhaler 1 by the user. First, during activation of a specific application, the user terminal 2 receives inhalation information from the medical inhaler 1 via the communication unit 25 in response to detection of the inhalation action of the medical inhaler 1. The connection for communication between the user terminal 2 and the medical inhaler 1 may be enabled either during the activation of the specific application or constantly, or may be enabled intermittently based on the activation of the medical inhaler 1.

Next, the user terminal 2 transmits the log information including at least the inhalation information to the information processing server 3 via the communication unit 25. The user terminal 2 may transmit the log information to the information processing server 3 in real time in response to acquisition of the inhalation information.

Although an example in which the user terminal 2 acquires inhalation information based on activation of a specific application has been described, the present invention is not limited thereto. The user terminal 2 may acquire the inhalation information irrespective of activation of a specific application.

The user terminal 2 may receive temperature information during activation of the medical inhaler 1 via the communication unit 25 in response to the detection of the inhalation action of the medical inhaler 1. The user terminal 2 may receive voltage information during activation of the medical inhaler 1 via the communication unit 25 in response to detection of the inhalation action of the medical inhaler 1. The user terminal 2 may receive biometric data while the user is using the medical inhaler 1 via the communication unit 25. The period during which the user is using the medical inhaler 1 corresponds to the period during which the medical inhaler 1 is activated. The log information may include the temperature information, voltage information, or biometric data. The log information may include medicament information of a medicament included in the aerosol source being used in the medical inhaler 1. The medicament information indicates a medicament name or a medicament ID. The medicament information may be input by the user from the input unit 23 of the user terminal 2. The medicament information may be acquired by the user terminal 2 from the medical inhaler 1.

The log information may include user information of a user who possesses the medical inhaler 1. The user information is information unique to a user who possesses the user terminal 2. For example, the user information is a preset user ID, user's attribute information, user's medical-related information, and the like. The user ID is an identifier uniquely assigned to the user terminal 2. The attribute information is information indicating attributes of the user, such as sex, age, and physique. The medical-related information is information indicating a symptom, a treatment method, a medication method, and the like of the user. The attribute information and the medical-related information may be input from the input unit 23 by the user. The attribute information and the medical-related information may be suitably updated. The user information is stored in the storage unit 22.

The log information may include type information indicating the type of the medical inhaler 1. The type information is, for example, information on a model number or the like of the medical inhaler 1. The type information is stored in the storage unit 22. The log information may include other information.

The way of transmitting the information included in the log information is a design matter, and each piece of information may be transmitted separately.

A hardware configuration of the user terminal 2 is not limited to the above-described configuration. The above-described constituent elements of the user terminal 2 may be suitably omitted or changed, and a new constituent element may be added to the user terminal 2.

### «Configuration Example of Information Processing Server»

FIG. 5 is a block diagram schematically showing a configuration example of an information processing server according to an embodiment of the present invention.

The information processing server 3 includes a control unit 31, a storage unit 32, and a communication unit 33. These units are mutually connected via a bus line.

The control unit 31 controls the entire operation of the information processing server 3 in accordance with programs stored in the storage unit 32. The control unit 31 is configured of, for example, electronic circuitry such as a processor. The processor is, for example, a CPU or the like. By executing the programs stored in the storage unit 32, the control unit 31 realizes the respective units that will be discussed later, and executes a variety of operations.

The storage unit 32 is configured of a main storage and an auxiliary storage, similarly to the above-described storage unit 22. The storage unit 32 stores programs for causing a control unit 31 to implement the respective units that will be discussed later. The storage unit 32 stores a log information DB 321, an atomization amount data DB 322, a denaturation data DB 323, a particle size data DB 324, and a delivery rate DB 325.

The log information DB 321 is a database that stores data based on log information transmitted from the user terminal 2 to the information processing server 3. The log information DB 321 is updated every time the information processing server 3 receives log information from the user terminal 2.

The atomization amount data DB 322 is a database that stores information indicating an atomization amount per unit volume of an inhalation volume [ml] for each of a plurality of medicaments. The atomization amount of the medicament per unit volume of the inhalation volume is, for example, an amount of the atomized medicament included in a unit volume (for example, 1 [ml]) of the inhalation volume of the user. The atomization amount of the medicament per unit volume of the inhalation volume is stored for each of the plurality of medicaments.

The atomization amount data DB 322 may be a database that stores information indicating atomization amounts of a plurality of medicaments per unit time. The atomization amount of the medicament per unit time indicates the amount of the medicament atomized per unit time. The atomization amount data DB 322 stores, for example, a record in which a "medicament name" or a "medicament ID" for identifying each medicament is associated with an "atomization amount per unit time" of each medicament according to the temperature. The data indicating the "atomization amount per unit time" of each medicament according to the temperature corresponds to the first atomization amount data. The atomization amount data DB 322 stores, for example, a record in which a "medicament name" or "medicament ID" is associated with an "atomization amount per unit time" of each medicament according to a voltage of the heating unit 121. The data indicating the "atomization amount per unit time" according to the voltage of each medicament corresponds to the second atomization amount data. The atomization amount data DB 322 may be stored in advance in the storage unit 32, or downloaded from a server (not illustrated) to the information processing server 3 via the network N. The atomization amount data DB 322 may be suitably updated.

The denaturation data DB 323 is a database that stores information indicating thermal denaturation rates of a plurality of medicaments according to the temperature. The thermal denaturation rate indicates a rate at which protein of each medicament may be thermally denatured according to the temperature. The denaturation data DB 323 stores, for example, a record in which a "medicament name" or a "medicament ID" for identifying each medicament is associated with a "thermal denaturation rate" of each medicament according to the temperature. The data indicating the "thermal denaturation rate" of each medicament according to the temperature corresponds to the denaturation data. The denaturation data DB 323 may be stored in advance in the storage unit 32, or downloaded from a server (not illustrated) to the information processing server 3 via the network N. The denaturation data DB 323 may be suitably updated. A configuration example of the denaturation data DB 323 will be described later.

The particle size data DB 324 is a database that stores information indicating particle sizes of a plurality of medicaments according to a temperature. Particle size refers to the average particle size (diameter) of the atomized medicament. The particle size data DB 324 stores, for example, a record in which a "medicament name" or a "medicament ID" for identifying each medicament is associated with a "particle size" of each medicament according to the temperature. The data indicating the "particle size" of each medicament according to the temperature corresponds to particle size data. The particle size data DB 324 may be stored in advance in the storage unit 32, or downloaded from a server (not illustrated) to the information processing server 3 via the network N. The particle size data DB 324 may be suitably updated. A configuration example of the particle size data DB 324 will be described later.

The delivery rate DB 325 is a database that stores information indicating delivery rates of a plurality of medicaments according to the particle size. The delivery rate indicates a rate at which the medicament reaches the user's body (for example, lungs). The delivery rate DB 325 stores, for example, a record in which a "medicament name" or a "medicament ID" for identifying each medicament is associated with a "delivery rate" of each medicament according to the particle size. The data indicating the "delivery rate" of each medicament according to the particle size corresponds to the delivery rate data. The delivery rate DB 325 may be stored in advance in the storage unit 32, or downloaded from a server (not illustrated) to the information processing server 3 via the network N. The delivery rate DB 325 may be suitably updated. A configuration example of the delivery rate DB 325 will be described later.

The communication unit 33 is configured of one or more communication interfaces that enable communications compatible with a given wireless communication standard to be performed. The communication unit 33 includes one or more communication interfaces capable of performing communication between the information processing server 3 and the user terminal 2 via the network N as described above.

A hardware configuration of the information processing server 3 is not limited to the above-described configuration. The above-described constituent elements of the information processing server 3 may be suitably omitted or changed, and a new constituent element may be added to the information processing server 3.

Each of the units realized by the above-described control unit 31 will be described.

The control unit 31 realizes a first acquisition unit 311, a first calculation unit 312, a second acquisition unit 313, a second calculation unit 314, and an output unit 315. It can also be said that each unit is realized by processing circuitry such as a processor included in the control unit 31. Each unit can also be referred to as each function. Each unit described below can be read as the control unit 31 or processing circuitry such as a processor.

The first acquisition unit 311 acquires inhalation information relating to inhalation by the user from the medical inhaler 1 based on the inhalation by the user of the medicament to be atomized by the medical inhaler 1. For example, the first acquisition unit 311 acquires information relating to an inhalation volume [ml] of a single inhalation by the user from the user terminal 2 connected to the medical inhaler 1 via the communication unit 33. A single inhalation refers to the start to end of a single inhalation action by the user. The first acquisition unit 311 may acquire information relating to an inhalation volume [ml/sec] by the user per unit time from the user terminal 2 connected to the medical inhaler 1 via the communication unit 33. The first acquisition unit 311 acquires inhalation information for a single inhalation by the user. The first acquisition unit 311 may acquire, for example, inhalation information based on the start or end of a single inhalation by the user from the user terminal 2 connected to the medical inhaler 1 via the communication unit 33. The first acquisition unit 311 acquires temperature information indicating a temperature of the heating unit 121 or voltage information indicating a voltage of the heating unit 121 from the medical inhaler 1. The first acquisition unit 311 acquires biometric data of the user while the user is using the medical inhaler 1.

The first calculation unit 312 may calculate an inhalation volume per inhalation of the user based on the inhalation information acquired by the first acquisition unit 311. If acquiring the information relating to an inhalation volume [ml/sec] per unit time of a single inhalation by the user, the first calculation unit 312 may calculate a inhalation volume [ml] of a single inhalation based on the time [sec] spent in a single inhalation (the time from the start to the end of a single inhalation action by the user).

Based on the inhalation information acquired by the first acquisition unit 311, the first calculation unit 312 calculates a total inhalation volume by summing inhalation volumes of each inhalation. The inhalation volume is an example of information relating to inhalation. The total inhalation volume is the sum of inhalation volumes for a series of consecutive inhalations. A series of consecutive inhalations includes inhalations in which the time between two consecutive inhalations is less than a certain time. A series of consecutive inhalations ends when a certain period of time has elapsed since the end information or execution information was received. The total inhalation volume is an example of total inhalation information.

The first calculation unit 312 may calculate a total inhalation time by summing inhalation times spent in each inhalation by the user based on the inhalation information acquired by the first acquisition unit 311. Here, the inhalation time indicates a temporal length of a single inhalation. The inhalation time is an example of information relating to inhalation. The total inhalation time is the sum of inhalation times spent for a series of consecutive inhalations. A series of consecutive inhalations includes inhalations in which the time between two consecutive inhalations is less than a certain time. A series of consecutive inhalations ends when a certain period of time has elapsed since the end information or execution information was received. The total inhalation time is an example of total inhalation information.

In the first example, the first calculation unit 312 calculates the total inhalation time by obtaining an inhalation time of a single inhalation based on start information and end information and summing inhalation times spent in each inhalation. In the second example, the first calculation unit 312 calculates the total inhalation time by obtaining an inhalation time of a single inhalation based on inhalation time information and summing inhalation times spent in each inhalation. In the third example, the first calculation unit 312 calculates the total inhalation time by counting the number of inhalation executions by the user based on execution information, and based on the number of executions and average inhalation time information stored in the storage unit 32. The execution information is, for example, start information or end information. The number of executions is, for example, the number of times the execution information is received. If the information processing server 3 receives one of the start information or the end information as the execution information, the number of executions is the number of times that the start information or the end information is received. If the information processing server 3 receives both the start information and the end information as the execution information, the number of executions is the number of times that either the start information or the end information is received. The average inhalation time information may indicate an average inhalation time per a single inhalation by a general user, or may be obtained by calculating an average inhalation time per a single inhalation by a user from a past history of the user of the medical inhaler 1. The average inhalation time information may be preset or suitably updated.

The second acquisition unit 313 acquires the atomization amount of the medicament per unit volume of the inhalation volume of the user. The second acquisition unit 313, for example, refers to the atomization amount data DB 322, and acquires the atomization amount of the medicament per unit volume of the inhalation volume of the user, based on the first atomization amount data and the temperature information, or the second atomization amount data and the temperature information, per unit volume of the inhalation volume. The atomization amount of the medicament per unit volume of the inhalation volume of the user is an example of atomization information relating to the atomization amount of the medicament.

The second calculation unit 314 calculates an intake amount of the medicament by the user by using the total inhalation volume calculated by the first calculation unit 312 and the atomization amount of the medicament per unit volume of the inhalation volume of the user acquired by the second acquisition unit 313.

The second acquisition unit 313 may acquire the atomization amount of the medicament per unit time. For example, the second acquisition unit 313 may refer to the atomization amount data DB 322, and acquire the atomization amount per unit time based on the first atomization amount data and the temperature information, or the second atomization amount data and the voltage information. The atomization amount of the medicament per unit time is an example of atomization information relating to the atomization amount of the medicament.

The second calculation unit 314 may calculate an intake amount of the medicament by the user by using the total inhalation time calculated by the first calculation unit 312 and the atomization amount per unit time acquired by the second acquisition unit 313.

In one example, the second calculation unit 314 can calculate an intake amount of the medicament by additionally using a non-denaturation rate of a medicament. The non-denaturation rate is a rate at which thermal denaturation of each medicament is predicted not to occur which is calculated based on a thermal denaturation rate of the protein of each medicament according to the temperature. In this example, the second calculation unit 314 obtains the non-denaturation rate of the medicament based on the temperature of the heating unit 121 of the medical inhaler 1 and the denaturation data stored in the denaturation data DB 323. The second calculation unit 314 calculates the intake amount of the medicament by using the obtained non-denaturation rate of the medicament.

In another example, the second calculation unit 314 may calculate an intake amount of the medicament by additionally using a delivery rate. In this example, the second calculation unit 314 obtains a particle size of the medicament atomized by the medical inhaler 1 based on the temperature of the heating unit 121 of the medical inhaler 1 and the particle size data stored in the particle size data DB324. The second calculation unit 314 obtains the delivery rate corresponding to the temperature of the heating unit 121 based on the particle size of the medicament atomized by the medical inhaler 1 and the delivery rate data stored in the delivery rate DB 325. The second calculation unit 314 calculates the intake amount of the medicament by using the acquired delivery rate. The calculation of the intake amount of the medicament by the second calculation unit 314 may use both the non-denaturation rate and the delivery rate of the medicament.

Furthermore, the second calculation unit 314 determines whether or not the intake amount of the medicament exceeds a reference amount. The reference amount is, for example, a dose of a medicament in accordance with the user's treatment method. The reference amount may be a single dose or a daily dose. The reference amount may be preset or suitably updated. The second calculation unit 314 calculates a remaining intake amount up to the reference amount in response to the intake amount of the medicament not exceeding the reference amount. Furthermore, the second calculation unit 314 calculates a remaining number of inhalations based on the remaining intake amount. The remaining number of inhalations corresponds to the allowable number of inhalations. The allowable number of inhalations is, for example, the number of inhalations allowed until the intake amount reaches the reference amount.

The output unit 315 outputs information relating to the intake amount of the medicament calculated by the second calculation unit 314. The information relating to the intake amount of the medicament includes some or all of information indicating the intake amount of the medicament, information indicating the remaining intake amount, and information indicating the remaining number of inhalations. The output unit 315 outputs information indicating the intake amount of the medicament calculated by the second calculation unit 314. The output unit 315 outputs information indicating the remaining intake amount calculated by the second calculation unit 314. The output unit 315 outputs information indicating the remaining number of inhalations calculated by the second calculation unit 314. Herein, "output" includes transmitting information to the user terminal 2 or an arbitrary terminal, or displaying information.

Furthermore, in one example, the output unit 315 outputs a stop instruction for the heating unit 121 in response to the second calculation unit 314 determining that the intake amount of the medicament exceeds the reference amount. In another example, the output unit 315 outputs a stop instruction to stop the heating unit 121 in response to the medical inhaler 1 detecting inhalations corresponding to the remaining number of inhalations. Here, "output" includes transmitting a stop instruction to the user terminal 2 or transmitting a stop instruction to the medical inhaler 1 via the user terminal 2.

Furthermore, the output unit 315 outputs the information relating to the intake amount and the biometric data acquired by the first acquisition unit 311 to the terminal. Here, "output" includes transmitting information to an arbitrary terminal or displaying information.

### <<Configuration Example of Denaturation Data DB>>

FIG. 5 is a diagram schematically showing a configuration example of a denaturation data DB according to the embodiment of the present invention.

The denaturation data DB 323 includes a record constituting data in which a "medicament name" and a "thermal denaturation rate" according to the temperature are associated with each other. For example, for "medicament A", it is shown that the thermal denaturation rate is "30%" when the temperature is less than 50 degrees, the thermal denaturation rate is "30%" when the temperature is 50 degrees or more and less than 100 degrees, and the thermal denaturation rate is "30%" when the temperature is 100 degrees or more and less than 200 degrees. For "medicament B", it is shown that the thermal denaturation rate is "50%" when the temperature is less than 50 degrees, the thermal denaturation rate is "80%" when the temperature is 50 degrees or more and less than 100 degrees, and the thermal denaturation rate is "100%" when the temperature is 100 degrees or more and less than 200 degrees. The thermal denaturation rate is not limited to being associated with a temperature range. The thermal denaturation rate may be associated with each temperature value on a one-to-one basis.

### «Configuration Example of Particle Size Data DB»

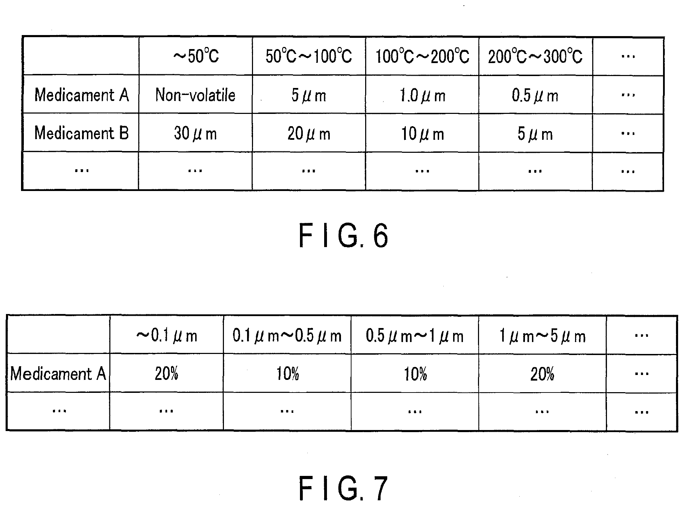

FIG. 6 is a diagram schematically showing a configuration example of a particle size data DB according to the embodiment of the present invention.

The particle size data DB 324 includes a record constituting data in which a "medicament name" and a "particle size" of each atomized medicament according to the temperature are associated with each other. For example, for "medicament A", it is shown that it is "non-volatile" when the temperature is less than 50 degrees, the particle size is "5 µm" when the temperature is 50 degrees or more and less than 100 degrees, the particle size is "1.0 µm" when the temperature is 100 degrees or more and less than 200 degrees, and the particle size is "0.5 µm" when the temperature is 200 degrees or more and less than 300 degrees. For "medicament B", it is shown that the particle size is "30 µm" when the temperature is less than 50 degrees, the particle size is "20 µm" when the temperature is 50 degrees or more and less than 100 degrees, the particle size is "10 µm" when the temperature is 100 degrees or more and less than 200 degrees, and the particle sizes "5 µm" when the temperature is 200 degrees or more and less than 300 degrees. The particle size is not limited to being associated with a temperature range. The particle size may be associated with each temperature value on a one-to-one basis.

### <<Configuration Example of Delivery Rate DB>>

FIG. 7 is a diagram schematically showing a configuration example of a delivery rate DB 325 according to the embodiment of the present invention.

The delivery rate DB 325 includes a record constituting data in which a "medicament name" and a "delivery rate" to a specific organ of the body according to the particle size are associated with each other. For example, for "medicament A", it is shown that the delivery rate is "20%" when the particle size is less than 0.1 µm, the delivery rate is "10%" when the particle size is 0.1 µm or more and less than 0.5 µm, the delivery rate is "10%" when the particle size is 0.5 µm or more and less than 1 µm, and the delivery rate is "20%" when the particle size is 1 µm or more and less than 5 µm. The delivery rate is not limited to being associated with a particle size range. The delivery rate may be associated with each particle size on a one to-one basis.

### <<Example of Processing by Information Processing Server>>

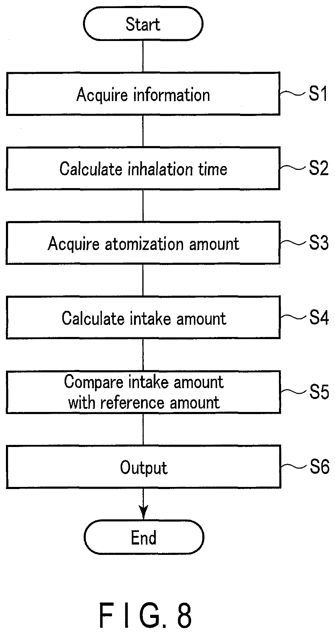

FIG. 8 is a flowchart showing an example of processing by the information processing server according to the embodiment of the present invention.

The processing procedure described below is merely an example, and each processing may be changed as much as possible. In addition, in the processing procedure described below, steps can be omitted, replaced, and added as appropriate according to the embodiment.

The first acquisition unit 311 acquires inhalation information from the medical inhaler 1 based on inhalation by the user of the medicament to be atomized by the medical inhaler 1 (step S1). At step S1, the first acquisition unit 311 acquires, for example, log information including inhalation information from the user terminal 2 connected to the medical inhaler 1 via the communication unit 33.

In a first example, the first acquisition unit 311 acquires inhalation information from the user terminal 2 based on the start or end of a single inhalation by the user (Example 1). In this example, the inhalation information includes start information or end information for a single inhalation. The first acquisition unit 311 acquires the start information and end information.

In the second example, the first acquisition unit 311 acquires inhalation information for a single inhalation by the user from the user terminal (Example 2). In this example, the inhalation information includes inhalation time information indicating the length of inhalation time for a single inhalation. The first acquisition unit 311 acquires the inhalation time information for a single inhalation.

In the third example, the first acquisition unit 311 acquires execution information indicating execution of a single inhalation by the user (Example 3). In this example, the execution information is information indicating the start or end of inhalation per inhalation. In this example, the inhalation information includes at least one of start information or end information for a single inhalation. The first acquisition unit 311 may acquire the start information or end information for a single inhalation as the execution information. Alternatively, the first acquisition unit 311 may acquire both the start information and the end information for a single inhalation as the execution information.

The first acquisition unit 311 can acquire temperature information or voltage information from the medical inhaler 1. The first acquisition unit 311 acquires, for example, log information including the temperature information or voltage information from the user terminal 2 connected to the medical inhaler 1 via the communication unit 33. The first acquisition unit 311 can acquire biometric data of the user while the user is using the medical inhaler 1. The first acquisition unit 311 acquires, for example, log information including biometric data from the user terminal 2 connected to the medical inhaler 1 via the communication unit 33.

The first calculation unit 312 calculates a total inhalation time by summing inhalation times spent in each inhalation by the user based on the inhalation information acquired by the first acquisition unit 311 (step S2). In Example 1 described above, the first calculation unit 312 calculates the total inhalation time by obtaining the inhalation time spent in the single inhalation based on the start information and the end information and summing the inhalation times spent in each inhalation. In this example, the first calculation unit 312 obtains the time from the start to the end based on the time information included in the log information for one set of the start information and the end information. For example, the first calculation unit 312 obtains the time from the start to the end based on a difference between the time indicated by the time information corresponding to the start information and the time indicated by the time information corresponding to the end information. The time from start to end based on the set of start information and end information is the inhalation time spent in a single inhalation. The first calculation unit 312 calculates the total inhalation time by summing the times obtained for each set of the start information and the end information. According to Example 1, since the processing of counting the time in the medical inhaler 1 is not required, the number of processing steps of the medical inhaler 1 can be reduced.

In Example 2 described above, the first calculation unit 312 calculates the total inhalation time by obtaining the inhalation time spent in the single inhalation based on the inhalation time information and summing the inhalation times spent in each inhalation. For example, the first calculation unit 312 obtains the inhalation time indicated by the inhalation time information. The first calculation unit 312 calculates the total inhalation time by summing the inhalation times obtained for each inhalation time information. According to Example 2, since counting the time in the information processing server 3 is not required, the number of processing steps of the information processing server 3 can be reduced.

In Example 3 described above, the first calculation unit 312 calculates the total inhalation time by counting the number of inhalation executions by the user based on the execution information, and based on the number of executions and the average inhalation time information stored in the storage unit 32,. For example, if the execution information is either the start information or the end information, the first calculation unit 312 counts the number of times that the start information or the end information is received, and obtains the number of executions. If the execution information is both the start information and the end information, the first calculation unit 312 counts the number of times that either the start information or the end information is received, and obtains the number of executions. The first calculation unit 312 calculates the total inhalation time based on a product of the obtained number of executions and the average inhalation time indicated by the average inhalation time information. According to Example 3, since the processing of counting the time in the medical inhaler 1 is not required, the number of processing steps of the medical inhaler 1 can be reduced.

The second acquisition unit 313 acquires the atomization amount of the medicament per unit time (step S3). At step S3, the second acquisition unit 313 acquires, for example, the atomization amount per unit time based on the first atomization amount data and the temperature information. In this example, the second acquisition unit 313 acquires the first atomization amount data associated with the medicament information stored in the log information DB 321 from the atomization amount data DB 322. The second acquisition unit 313 acquires the temperature information from the storage unit 32. The second acquisition unit 313 refers to the first atomization amount data, and acquires the atomization amount per unit time according to the temperature of the heating unit 121 indicated by the temperature information. The temperature information may be acquired from the log information DB 321 or may be stored in the storage unit 32 in advance. If the temperature information is acquired from the log information DB 321, the second calculation unit 314 acquires, for example, the temperature of the heating unit 121 based on the temperature information acquired by the first acquisition unit 311. If the temperature of the heating unit 121 is set in advance, the temperature information is stored in advance in the storage unit 32. In this case, the second calculation unit 314 acquires the temperature of the heating unit 121 based on the temperature information stored in the storage unit 32.

In another example, the second acquisition unit 313 acquires the atomization amount per unit time based on the second atomization amount data and the voltage information. In this example, the second acquisition unit 313 acquires the second atomization amount data associated with the medicament information stored in the log information DB 321 from the atomization amount data DB 322. The second acquisition unit 313 acquires the voltage information from the storage unit 32. The second acquisition unit 313 refers to the second atomization amount data, and acquires the atomization amount per unit time corresponding to the voltage of the heating unit 121 indicated by the voltage information. The voltage information may be acquired from the log information DB 321, or may be stored in the storage unit 32 in advance. If the voltage information is acquired from the log information DB 321, the second calculation unit 314 acquires, for example, the voltage of the heating unit 121 based on the voltage information acquired by the first acquisition unit 311. If the voltage of the heating unit 121 is set in advance, the voltage information is stored in advance in the storage unit 32. In this case, the second calculation unit 314 acquires the voltage of the heating unit 121 based on the voltage information stored in the storage unit 32.

The second calculation unit 314 calculate an intake amount of the medicament by the user by using the total inhalation time calculated by the first calculation unit 312 and the atomization amount per unit time acquired by the second acquisition unit 313 (step S4). At step S4, the second calculation unit 314 calculates, for example, the intake amount based on a product of the total inhalation time and the atomization amount per unit time. According to this example, since the information processing server 3 can calculate the intake amount based on the time of inhalation of the medicament by the user and the atomization amount per unit time, there is little possibility that the amount of the medicament atomized in the air is included in the intake amount. Therefore, the information processing server 3 can more accurately calculate the intake amount of the medicament by the user, and can improve the accuracy of the calculation of the intake amount.

In one example, the second calculation unit 314 may calculate the intake amount of the medicament by additionally using a non-denaturation rate of a medicament. In this example, first, the second calculation unit 314 acquires temperature information indicating the temperature of the heating unit 121 of the medical inhaler 1 from the storage unit 32. The temperature information may be acquired from the log information DB 321 or may be stored in the storage unit 32 in advance. The second calculation unit 314 acquires the denaturation data associated with the medicament information stored in the log information DB 321 from the denaturation data DB 323. Next, the second calculation unit 314 acquires the denaturation rate of the medicament corresponding to the temperature of the heating unit 121 based on the denaturation data. The second calculation unit 314 calculates the non-denaturation rate of the medicament from the acquired denaturation rate of the medicament. For example, if the denaturation rate is 30%, the non-denaturation rate is the remaining 70%. Next, the second calculation unit 314 calculates the intake amount based on a product of the total inhalation time, the atomization amount per unit time, and the non-denaturation rate of the medicament. According to this example, since the information processing server 3 can calculate the intake amount in consideration of the denaturation rate of the medicament that varies depending on the temperature, it is possible to more accurately calculate the intake amount of the medicament of the user. Therefore, the information processing server 3 can further improve the accuracy of the calculation of the intake amount.

In another example, the second calculation unit 314 can calculate the intake amount of the medicament by additionally using the delivery rate. In this example, first, the second calculation unit 314 acquires temperature information indicating the temperature of the heating unit 121 of the medical inhaler 1 from the storage unit 32. The temperature information may be acquired from the log information DB 321 or may be stored in the storage unit 32 in advance. The second calculation unit 314 acquires the particle size data associated with the medicament information stored in the log information DB 321 from the particle size data DB 324. Next, the second calculation unit 314 acquires the particle size of the medicament atomized by the medical inhaler 1 corresponding to the temperature of the heating unit 121 based on the particle size data. Next, the second calculation unit 314 acquires the delivery rate data associated with the medicament information stored in the log information DB 321 from the delivery rate DB 325. The second calculation unit 314 acquires the delivery rate corresponding to the acquired particle size of the medicament based on the delivery rate data. The delivery rate acquired by the second calculation unit 314 corresponds to the temperature of the heating unit 121. The second calculation unit 314 calculates the intake amount of the medicament based on a product of the total inhalation time, the atomization amount per unit time, and the delivery rate. According to this example, since the information processing server 3 can calculate the intake amount in consideration of the particle size of the medicament that varies depending on the temperature and the delivery rate that varies depending on the particle size, it is possible to more accurately calculate the intake amount of the medicament of the user. Therefore, the information processing server 3 can further improve the accuracy of the calculation of the intake amount.

The second calculation unit 314 determines whether or not the intake amount of the medicament exceeds the reference amount (step S5). At step S5, the second calculation unit 314 calculates, for example, the remaining intake amount up to the reference amount in response to the intake amount of the medicament not exceeding the reference amount. The second calculation unit 314 may calculate the remaining number of inhalations based on the remaining intake amount. In this example, the second calculation unit 314 calculates the remaining number of inhalations by using the single inhalation time or the average inhalation time and the remaining intake amount. The step S5 can be omitted.

The output unit 315 outputs information relating to the intake amount based on the calculation by the second calculation unit 314 (step S6). If step S5 is omitted, the output unit 315 outputs the information indicating the intake amount of the medicament calculated by the second calculation unit 314 to the user terminal 2. The user terminal 2 displays the information received from the output unit 315 on the display unit 24 via the communication unit 25. The user terminal 2 displays the received information on the display unit 24 in a given display mode. According to this example, by notifying the user of the intake amount, the information processing server 3 can support the user to prevent an overdose of the medicament.

If the processing of step S5 is performed, in one example, the output unit 315 outputs information indicating the remaining intake amount calculated by the second calculation unit 314 to the user terminal 2. In another example, the output unit 315 outputs information indicating the remaining number of inhalations calculated by the second calculation unit 314 to the user terminal 2. The user terminal 2 displays the information received from the output unit 315 on the display unit 24 via the communication unit 25. The user terminal 2 displays the received information on the display unit 24 in a given display mode. According to this example, by notifying the user of the remaining intake amount or the remaining number of inhalations, the information processing server 3 makes it easy for the user to grasp the intake amount and can further support the user to prevent an overdose of the medicament.

Furthermore, the output unit 315 outputs a stop instruction of the heating unit 121 of the medical inhaler 1 in response to the second calculation unit 314 determining that the intake amount of the medicament exceeds the reference amount. In one example, the output unit 315 outputs a stop instruction in response to the second calculation unit 314 determining that the remaining intake amount exceeds the reference amount. In another example, the output unit 315 outputs a stop instruction for the heating unit 121 in response to detection of inhalations corresponding to the remaining number of inhalations. In this example, the second calculation unit 314 counts the number of inhalations based on the inhalation information acquired by the first acquisition unit 311. The output unit 315 may output a stop instruction for the heating unit 121 at a point in time when inhalations corresponding to the remaining number of inhalations are counted. According to this example, the information processing server 3 can stop the discharge of the medicament without requiring a user's judgment, and can prevent an overdose of the medicament by the user.

In still another example, the output unit 315 outputs a stop instruction to stop the heating unit of the medical inhaler 1 in response to the medical inhaler 1 detecting inhalations corresponding to the remaining number of inhalations. In this case, the output unit 315 outputs information on the remaining number of inhalations to the medical inhaler 1. The medical inhaler 1 stops the heating unit in response to detection of inhalations corresponding to the remaining number of inhalations. According to this example, since the medical inhaler 1 itself stops the heating unit 121 and stops the discharge of the medicament, the information processing server 3 can prevent an overdose of the medicament by the user. In addition, the process of counting the remaining number of inhalations in the information processing server 3 can be omitted, and the number of processing steps in the information processing server 3 can be reduced.

The output unit 315 may output the information relating to the intake amount and the biometric data acquired by the first acquisition unit 311 to a terminal different from the user terminal 2. The output unit 315 may output one or both of the information relating to the intake amount and the biometric data. In addition, the information relating to the intake amount and the biometric data may be separately output. According to this example, a terminal different from the user terminal 2 can output an operation instruction to the medical inhaler 1 based on an operation by a medical worker or the like. According to this example, since the information processing server 3 can present the information relating to the intake amount and the biometric data to the terminal possessed by the medical worker or the like, it is possible to control the medicament intake by the medical worker or the like.

Furthermore, the output unit 315 may output inhalation history information to the user terminal 2 or a terminal different from the user terminal 2 based on the log information acquired by the first acquisition unit 311. The inhalation history information may include inhalation information, inhalation time, inhalation place, biometric data at the time of inhalation, and the like.

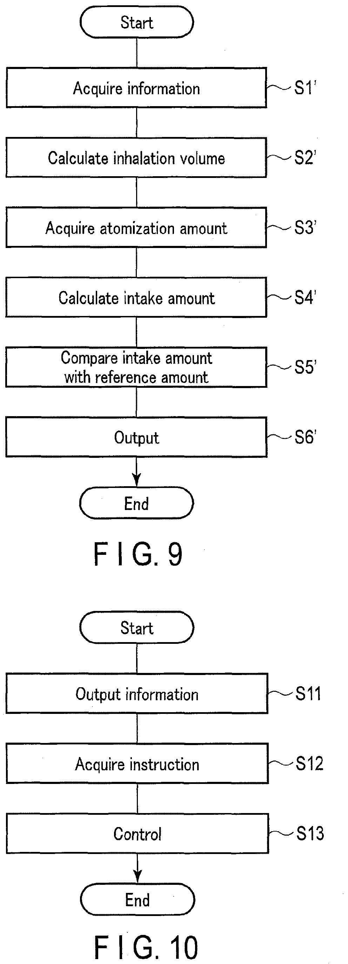

FIG. 9 is a flowchart showing an example of processing by the information processing server according to the embodiment of the present invention. The processing example of FIG. 9 is a processing example in a case where the information processing server acquires, as inhalation information, information relating to an inhalation volume [ml] of a single inhalation by the user, or information relating to an inhalation volume [ml] a single inhalation by the user.

The first acquisition unit 311 acquires inhalation information from the medical inhaler 1 based on inhalation by the user of the medicament to be atomized by the medical inhaler 1 (step S1'). The first acquisition unit 311 acquires information relating to the inhalation volume [ml] of a single inhalation by the user from the user terminal 2. The first acquisition unit 311 may acquire information relating to an inhalation volume [ml/sec] of the user per unit time from the user terminal 2 connected to the medical inhaler 1 via the communication unit 33.

If acquiring the information relating to an inhalation volume [ml/sec] per unit time of a single inhalation by the user, the first calculation unit 312 may calculate a inhalation volume [ml] of a single inhalation based on the time [sec] of a single inhalation (the time from the start to the end of a single inhalation action by the user) (step S2').

Based on the inhalation information acquired by the first acquisition unit 311, the first calculation unit 312 calculates a total inhalation volume by summing inhalation volumes of each inhalation (step S2'). If acquiring the information relating to the inhalation volume [ml] of the single inhalation by the user, the first calculation unit 312 calculates a total inhalation volume which is a sum of inhalation volumes for a series of consecutive inhalations.

The second acquisition unit 313 acquires information indicating the atomization amount of the medicament per unit volume of the inhalation volume [ml] (step S3'). At step S3', the second acquisition unit 313 acquires, for example, the atomization amount per unit volume based on the first atomization amount data and the temperature information. In this example, the second acquisition unit 313 acquires the first atomization amount data associated with the medicament information stored in the log information DB 321 from the atomization amount data DB 322. The second acquisition unit 313 acquires the temperature information from the storage unit 32. The second acquisition unit 313 refers to the first atomization amount data, and acquires the atomization amount per unit volume corresponding to the temperature of the heating unit 121 indicated by the temperature information. The temperature information may be acquired from the log information DB 321 or may be stored in the storage unit 32 in advance. If the temperature information is acquired from the log information DB 321, the second calculation unit 314 acquires, for example, the temperature of the heating unit 121 based on the temperature information acquired by the first acquisition unit 311. If the temperature of the heating unit 121 is set in advance, the temperature information is stored in advance in the storage unit 32. In this case, the second calculation unit 314 acquires the temperature of the heating unit 121 based on the temperature information stored in the storage unit 32.

The second calculation unit 314 calculate an intake amount of the medicament by the user by using the total inhalation volume calculated by the first calculation unit 312 and the atomization amount per unit volume acquired by the second acquisition unit 313 (step S4). At step S4', the second calculation unit 314 calculates, for example, the intake amount based on a product of the total inhalation volume and the atomization amount per unit volume. According to this example, since the information processing server 3 can calculate the intake amount from the inhalation volume of the medicament inhaled by the user and the atomization amount per unit volume, there is little possibility that the amount of the medicament atomized in the air is included in the intake amount. Therefore, the information processing server 3 can more accurately calculate the intake amount of the medicament by the user, and can improve the accuracy of the calculation of the intake amount.

In one example, the second calculation unit 314 can calculate the intake amount of the medicament by additionally using a non-denaturation rate of the medicament. In this example, first, the second calculation unit 314 acquires temperature information indicating the temperature of the heating unit 121 of the medical inhaler 1 from the storage unit 32. The temperature information may be acquired from the log information DB 321 or may be stored in the storage unit 32 in advance. The second calculation unit 314 acquires the denaturation data associated with the medicament information stored in the log information DB 321 from the denaturation data DB 323. Next, the second calculation unit 314 acquires the denaturation rate of the medicament corresponding to the temperature of the heating unit 121 based on the denaturation data. The second calculation unit 314 calculates the non-denaturation rate of the medicament from the acquired denaturation rate of the medicament. For example, if the denaturation rate is 30%, the non-denaturation rate is the remaining 70%. Next, the second calculation unit 314 calculates the intake amount by a product of the total inhalation volume, the atomization amount per unit volume, and the non-denaturation rate of the medicament. According to this example, since the information processing server 3 can calculate the intake amount in consideration of the denaturation rate of the medicament that varies depending on the temperature, it is possible to more accurately calculate the intake amount of the medicament of the user. Therefore, the information processing server 3 can further improve the accuracy of the calculation of the intake amount.

In another example, the second calculation unit 314 may calculate the intake amount of the medicament by additionally using the delivery rate. In this example, first, the second calculation unit 314 acquires temperature information indicating the temperature of the heating unit 121 of the medical inhaler 1 from the storage unit 32. The temperature information may be acquired from the log information DB 321 or may be stored in the storage unit 32 in advance. The second calculation unit 314 acquires the particle size data associated with the medicament information stored in the log information DB 321 from the particle size data DB 324. Next, the second calculation unit 314 acquires the particle size of the medicament atomized by the medical inhaler 1 corresponding to the temperature of the heating unit 121 based on the particle size data. Next, the second calculation unit 314 acquires the delivery rate data associated with the medicament information stored in the log information DB 321 from the delivery rate DB 325. The second calculation unit 314 acquires the delivery rate corresponding to the acquired particle size of the medicament based on the delivery rate data. The delivery rate acquired by the second calculation unit 314 corresponds to the temperature of the heating unit 121. The second calculation unit 314 calculates the intake amount of the medicament based on a product of the total inhalation volume, the atomization amount per unit volume, and the delivery rate. According to this example, since the information processing server 3 can calculate the intake amount in consideration of the particle size of the medicament that varies depending on the temperature and the delivery rate that varies depending on the particle size, it is possible to more accurately calculate the intake amount of the medicament of the user. Therefore, the information processing server 3 can further improve the accuracy of the calculation of the intake amount.

Since S5' and S6' in FIG. 9 are similar processes to S5 and S6 in FIG. 8, respectively, a detailed description thereof will be omitted.

### <<Processing Example by Medical Inhaler>>

FIG. 10 is a flowchart showing an example of processing by the medical inhaler according to the embodiment of the present invention.

The processing procedure described below is merely an example, and each processing may be changed as much as possible. In addition, in the processing procedure described below, steps can be omitted, replaced, and added as appropriate according to the embodiment.

The output unit 1161 outputs the inhalation information relating to the inhalation of the medicament by the user through the mouthpiece 124 to the information processing server 3 (step S11). At step S11, the output unit 1161 outputs, for example, the inhalation information for the information processing server 3 acquired based on the detection of the inhalation action to the user terminal 2 via the communication unit 115.

The acquisition unit 1162 acquires an operation instruction of the heating unit 121 from the information processing server 3 as a response to the inhalation information output by the output unit 1161 (step S12). At step S12, the acquisition unit 1162 acquires, for example, a stop instruction for the heating unit 121 from the information processing server 3 via the user terminal 2. In another example, the acquisition unit 1162 acquires a temperature adjustment instruction for the heating unit 121 from the information processing server 3 via the user terminal 2. The acquisition unit 1162 may acquire a stop instruction to stop the heating unit 121 in response to the medical inhaler 1 detecting inhalations corresponding to the remaining number of inhalations via the user terminal 2. In this case, the acquisition unit 1162 acquires information on the remaining number of inhalations calculated by the information processing server 3 via the user terminal 2.

The atomization control unit 1163 instructs temperature adjustment or stopping of the heating unit 121 based on the operation instruction (step S13). At step S13, the atomization control unit 1163 increases or decreases the amount of power supplied to the heating unit 121 or stops the power supply to the heating unit 121 based on the operation instruction from the information processing server 3. According to this example, the medical inhaler 1 can stop the discharge of the medicament without requiring a user's judgment, and can prevent an overdose of the medicament by the user.

The atomization control unit 1163 may stop the heating unit 121 in response to detection of inhalations corresponding to the remaining number of inhalations. In this case, the atomization control unit 1163 counts the remaining number of inhalations with reference to the inhalation information or the like based on the information on the remaining number of inhalations acquired by the acquisition unit 1162. If the counted number of inhalations reaches the remaining number of inhalations, the atomization control unit 1163 stops the heating unit 121. According to this example, the medical inhaler 1 can control the discharge of the medicament without requiring a user's judgment, and can prevent an overdose of the medicament by the user. In addition, the process of counting the remaining number of inhalations in the information processing server 3 can be omitted, and the number of processing steps in the information processing server 3 can be reduced.

In a case where the aerosol source is atomized by means such as vibration other than heating, the medical inhaler 1 includes an element different from the heating unit 121 as an atomizing unit. In this case, the atomization control unit 1163 controls the operation of the atomizing unit.

The above-described embodiment can also be applied to various suction devices or inhalers such as a flavor inhaler. If the above-described embodiment is applied to the flavor inhaler, the storage unit 123 stores an aerosol source. The aerosol source is an example of a solution. The aerosol source may contain, for example, a tobacco derived or non-tobacco derived flavor component.

As an example, the flavor inhaler may include a flavor-imparting cartridge. In that case, the flavor-imparting cartridge is provided with a flavor source and the mouthpiece 124. The flavor source is a constituent element for imparting a flavor component to aerosol. The flavor source may contain a tobacco-derived or non-tobacco-derived flavor component. The air flowing in from the air inflow hole 181 in accordance with user inhalation is mixed with the aerosol produced by the heating unit 121, passes through the flavor source and is transported to the air outflow hole 182. When the mixed fluid of the aerosol and air passes through the flavor source, the flavor component contained in the flavor source is imparted to the aerosol.

Although the information processing server 3 has been described as an example of the information processing device according to the embodiment, the information processing device may be realized by the user terminal 2. In this case, the user terminal 2 may have the function of the information processing server 3 to implement the above-described embodiment.

The information processing device according to the embodiment may be realized by one device as described using the information processing server 3 as an example, or by a plurality of devices in which functions are distributed.

The program may be transferred in a state of being stored in the device, or may be transferred in a state of not being stored in the device. In the latter case, the program may be transferred via a network, or may be transferred in a state of being recorded in a recording medium. The recording medium is a non-transitory tangible medium. The recording medium is a computer-readable medium. The recording medium may be any medium that can store a program and can be read by a computer, such as a CD-ROM or a memory card, and may be in any form.

The present invention is not limited to the above-described embodiments, and can be modified in various manners in practice during implementation without departing from the gist of the invention. Moreover, the embodiments can be suitably combined; in that case, the combined advantages are obtained. Furthermore, the above-described embodiments include various inventions, and various inventions can be extracted by a combination selected from structural elements disclosed herein. For example, if the object of the invention is achieved and the advantages of the invention are attained even after some of the structural elements disclosed in connection with the embodiments are deleted, the structure made up of the resultant structural elements can be extracted as an invention.

### REFERENCE SIGNS LIST

1: medical inhaler, 2: user terminal, 3: information processing server, 10: information processing system, 21: control unit, 22, storage unit, 23: input unit, 24: display unit, 25: communication unit, 26: detection unit, 31: control unit, 32: storage unit, 33: communication unit, 110: power-supply unit, 111: power supply,112: sensor unit, 113: notification unit, 114: storage unit, 115: communication unit, 116: control unit, 120: cartridge, 121: heating unit, 122: liquid guide unit, 123: storage unit, 124: mouthpiece, 140: holding part, 141: internal space, 142: opening, 143: bottom portion, 144: heat-insulating part, 150: stick-shaped base material, 151: base material portion, 152: suction portion, 180: airflow path, 181: air inflow hole, 182: air outflow hole, 190: arrow, 311: first acquisition unit, 312: first calculation unit, 313: second acquisition unit, 314: second calculation unit, 315: output unit, 321: log information DB, 322: atomization amount data DB, 323: denaturation data DB, 324: particle size data DB, 325: delivery rate DB, 1161: output unit, 1162: acquisition unit, 1163: atomization control unit, N: network.

## Claims

1. An information processing device comprising:
a first acquisition unit configured to acquire, based on inhalation by a user of a medicament atomized by a medical device, inhalation information relating to inhalation by the user from the medical device;
a first calculation unit configured to calculate total inhalation information obtained by summing information relating to inhalation per inhalation by the user based on the inhalation information acquired by the first acquisition unit;
a second acquisition unit configured to acquire atomization information relating to an atomization amount of the medicament;
a second calculation unit configured to calculate an intake amount of the medicament by the user by using the total inhalation information calculated by the first calculation unit and the atomization information acquired by the second acquisition unit; and
an output unit configured to output information relating to the intake amount of the medicament calculated by the second calculation unit.

2. The information processing device according to claim 1, wherein
the inhalation information includes an inhalation volume of a single inhalation by the user,
the first calculation unit is configured to calculate a total inhalation volume by summing inhalation volumes of each inhalation by the user based on the inhalation volume of the single inhalation by the user acquired by the first acquisition unit,
the second acquisition unit is configured to acquire an atomization amount of the medicament per unit volume of the inhalation volume of the user, and
the second calculation unit is configured to calculate an intake amount of the medicament by the user by using the total inhalation volume calculated by the first calculation unit and the atomization amount of the medicament per unit volume acquired by the second acquisition unit.

3. The information processing device according to claim 1, wherein
the first calculation unit is configured to calculate a total inhalation time by summing inhalation times spent in each inhalation by the user based on the inhalation information acquired by the first acquisition unit,
the second acquisition unit is configured to acquire an atomization amount of the medicament per unit time, and
the second calculation unit is configured to calculate an intake amount of the medicament by the user by using the total inhalation time calculated by the first calculation unit and the atomization amount per unit time acquired by the second acquisition unit.

4. The information processing device according to claim 3, wherein
the inhalation information includes start information or end information for a single inhalation by the user,
the first acquisition unit is configured to acquire the inhalation information based on start or end of the single inhalation by the user, and
the first calculation unit is configured to calculate the total inhalation time by obtaining an inhalation time spent in the single inhalation based on the start information and the end information and summing inhalation times spent in each inhalation.

5. The information processing device according to claim 3, wherein
the inhalation information includes inhalation time information indicating an inhalation time spent in a single inhalation by the user,
the first acquisition unit is configured to acquire the inhalation information for the single inhalation by the user, and the first calculation unit is configured to calculate the total inhalation time by obtaining an inhalation time spent in the single inhalation based on the inhalation time information and summing inhalation times spent in each inhalation.

6. The information processing device according to claim 3, further comprising:
a storage unit configured to store average inhalation time information indicating an average inhalation time spent in a single inhalation by the user,
wherein the inhalation information includes execution information indicating execution of the single inhalation by the user,
the first acquisition unit is configured to acquire the inhalation information for the single inhalation by the user, and
the first calculation unit is configured to calculate the total inhalation time by counting a number of executions of inhalation by the user based on the execution information, and based on the number of executions and the average inhalation time information stored in the storage unit.

7. The information processing device according to claim 3, further comprising:
a storage unit configured to store first atomization amount data indicating an amount of the medicament atomized per unit time according to a temperature, or second atomization amount data indicating an amount of the medicament atomized per unit time according to a voltage of a heating unit of the medical device,
wherein the first acquisition unit is configured to acquire, from the medical device, temperature information indicating the temperature of the heating unit or voltage information indicating the voltage of the heating unit, and
the second acquisition unit is configured to acquire the atomization amount per unit time based on the first atomization amount data and the temperature information, or the second atomization amount data and the voltage information.

8. The information processing device according to any one of claims 1 to 7, further comprising:
a storage unit configured to store denaturation data relating to denaturation of the medicament according to a temperature,
wherein the second calculation unit is configured to acquire a non-denaturation rate of the medicament based on a temperature of a heating unit atomizing the medicament of the medical device and the denaturation data stored in the storage unit, and
the second calculation unit is configured to calculate an intake amount of the medicament by using the non-denaturation rate of the medicament.

9. The information processing device according to any one of claims 1 to 8, further comprising:
a storage unit configured to store particle size data relating to a particle size of the atomized medicament according to a temperature, and delivery rate data relating to a rate of delivery into a body according to a particle size,
wherein the second calculation unit is configured to acquire a particle size of the medicament atomized by the medical device based on a temperature of a heating unit atomizing the medicament of the medical device and the particle size data stored in the storage unit, acquire a delivery rate according to the temperature of the heating unit of the medical device based on the particle size of the medicament atomized by the medical device and the delivery rate data, and calculate an intake amount of the medicament using the acquired delivery rate.

10. The information processing device according to claim 8 or 9, wherein
the first acquisition unit is configured to acquire, from the medical device, temperature information indicating the temperature of the heating unit or voltage information indicating the voltage of the heating unit, and
the second calculation unit is configured to acquire the temperature of the heating unit based on the temperature information or the voltage information.

11. The information processing device according to claim 8 or 9, wherein
the storage unit is configured to store temperature information indicating the temperature of the heating unit, and
the second calculation unit is configured to acquire the temperature of the heating unit based on the temperature information stored in the storage unit.

12. The information processing device according to any one of claims 1 to 10, wherein
the second calculation unit is configured to determine whether the intake amount of the medicament exceeds a reference amount, and calculate a remaining intake amount up to the reference amount in response to the intake amount of the medicament not exceeding the reference amount, and
the output unit is configured to output information indicating the remaining intake amount calculated by the second calculation unit.

13. The information processing device according to any one of claims 1 to 10, wherein
the second calculation unit is configured to determine whether the intake amount of the medicament exceeds a reference amount, and
the output unit is configured to output a stop instruction for a heating unit of the medical device in response to the second calculation unit determining that the intake amount of the medicament exceeds the reference amount.

14. The information processing device according to any one of claims 1 to 10, wherein
the second calculation unit is configured to determine whether the intake amount of the medicament exceeds a reference amount, calculate a remaining intake amount up to the reference amount in response to the intake amount of the medicament not exceeding the reference amount, and calculate a remaining number of inhalations based on the remaining intake amount, and
the output unit is configured to output information indicating the remaining number of inhalations calculated by the second calculation unit.

15. The information processing device according to claim 14, wherein
the output unit is configured to output a stop instruction to stop a heating unit of the medical device in response to the medical device detecting inhalations corresponding to the remaining number of inhalations.

16. The information processing device according to any one of claims 1 to 15, wherein
the first acquisition unit is configured to acquire biometric data of the user while the user is using the medical device, and
the output unit is configured to output, to a terminal, the information relating to the intake amount and the biometric data acquired by the first acquisition unit.

17. An information processing method comprising:
acquiring, based on inhalation by a user of a medicament atomized by a medical device, inhalation information relating to inhalation by the user from the medical device;
calculating total inhalation information obtained by summing information relating to inhalation of each inhalation by the user based on the acquired inhalation information;
acquiring atomization information relating to an atomization amount of the medicament;
calculating an intake amount of the medicament by the user by using the calculated total inhalation information and the acquired atomization information; and
outputting information relating to the intake amount of the calculated medicament.

18. A program for causing a computer to implement:
a first acquisition unit configured to acquire, based on inhalation by a user of a medicament atomized by a medical device, inhalation information relating to inhalation by the user from the medical device;
a first calculation unit configured to calculate total inhalation information obtained by summing information relating to inhalation of each inhalation by the user based on the inhalation information acquired by the first acquisition unit;
a second acquisition unit configured to acquire atomization information relating to an atomization amount of the medicament;
a second calculation unit configured to calculate an intake amount of the medicament by the user by using the total inhalation information calculated by the first calculation unit and the atomization information acquired by the second acquisition unit; and
an output unit configured to output information relating to the intake amount of the medicament calculated by the second calculation unit.

19. An information processing device comprising:
a first acquisition unit configured to acquire, based on inhalation by a user of a solution atomized by an inhaler, inhalation information relating to inhalation by the user from the inhaler;
a first calculation unit configured to calculate total inhalation information obtained by summing information relating to inhalation of each inhalation by the user based on the inhalation information acquired by the first acquisition unit;
a second acquisition unit configured to acquire atomization information relating to an atomization amount of the solution;
a second calculation unit configured to calculate an intake amount of the solution by the user by using the total inhalation information calculated by the first calculation unit and the atomization information acquired by the second acquisition unit; and
an output unit configured to output information relating to the intake amount of the solution calculated by the second calculation unit.

20. A medical device comprising:
a storage unit configured to store a medicament;
a heating unit configured to heat the medicament;
an inhalation unit for inhalation of the medicament atomized by the heating unit;
an output unit configured to output, to an information processing device, inhalation information relating to inhalation of the medicament by the user via the inhalation unit;
an acquisition unit configured to acquire an operation instruction of the heating unit from the information processing device as a response to the inhalation information output by the output unit; and
a control unit configured to control stopping of the heating unit based on the operation instruction.

21. The medical device according to claim 20, wherein
the operation instruction includes a stop instruction for stopping the heating unit in response to the medical device detecting an inhalation of an allowable number of inhalations calculated by the information processing device, and
the control unit is configured to stop the heating unit based on the allowable number of inhalations of the medicament by the user.

22. An inhaler comprising:
a storage unit configured to store a solution;
a heating unit configured to heat the solution;
an inhalation unit for inhalation of the solution atomized by the heating unit;
an output unit configured to output, to an information processing device, inhalation information relating to inhalation of the solution by the user via the inhalation unit;
an acquisition unit configured to acquire an operation instruction of the heating unit from the information processing device as a response to the inhalation information output by the output unit; and
a control unit configured to control stopping of the heating unit based on the operation instruction.
